(19) Europäisches Patentamt
European Patent Office
Office européen des brevets

(11) EP 1 784 376 B1

(12) **EUROPEAN PATENT SPECIFICATION**

(45) Date of publication and mention of the grant of the patent:
**10.10.2012 Bulletin 2012/41**

(21) Application number: **06783682.5**

(22) Date of filing: **22.08.2006**

(51) Int Cl.:
***C07C 51/235*** *(2006.01)*

(86) International application number:
**PCT/KR2006/003286**

(87) International publication number:
**WO 2007/029929 (15.03.2007 Gazette 2007/11)**

(54) **METHOD OF PREPARING ORGANIC ACIDS FROM ALDEHYDE COMPOUNDS BY MEANS OF LIQUID PHASE OXIDATION REACTION**

VERFAHREN ZUR HERRSTELLUNG VON ORGANISCHEN SAUREN AUS ALDEHYD VERBINDUNGEN DURCH OXIDATIONSREAKTION IN DER FLUSSIGPHASE

PROCEDE DE PREPARATION D'ACIDES ORGANIQUES A PARTIR DE COMPOSES D'ALDEHYDE AU MOYEN D'UNE REACTION D'OXYDATION A PHASE LIQUIDE

(84) Designated Contracting States:
**DE FR NL**

(30) Priority: **07.09.2005 KR 20050083417**

(43) Date of publication of application:
**16.05.2007 Bulletin 2007/20**

(73) Proprietor: **LG Chem, Ltd.**
**Seoul 150-721 (KR)**

(72) Inventors:
- **KO, Dong-hyun**
**Daejeon 305-350 (KR)**
- **LEE, Sang-gi**
**Daejeon 305-728 (KR)**
- **KWON, O-hak**
**Daejeon 302-739 (KR)**
- **MOON, Ji-joong**
**Daejeon 305-738 (KR)**
- **KIM, Dae-chul**
**Daejeon 302-740 (KR)**
- **CHOI, Jae-hui**
**Gyeonggi-do 431-070 (KR)**
- **HONG, Bog-ki**
**Daejeon 305-738 (KR)**
- **EOM, Sung-shik**
**Daejeon 305-340 (KR)**

(74) Representative: **Goddar, Heinz J.**
**Boehmert & Boehmert**
**Pettenkoferstrasse 20-22**
**80336 München (DE)**

(56) References cited:
**WO-A1-2004/029004 US-A- 5 731 101**

- **DATABASE WPI Week 200518 Thomson Scientific, London, GB; AN 2005-164021 XP002546004 & CN 1 544 410 A (INST PROCESS ENG CHINESE ACAD SCI) 10 November 2004 (2004-11-10)**
- **HOWARTH J.: 'Oxidation of aromatic aldehydes in the ionic liquid [bmim]PF6' TETRAHEDRON LETTERS vol. 41, 2000, pages 6627 - 6629, XP004215829**
- **HSU J.-C. ET AL.: 'Baylis-Hillman reaction in [bdmim][PF6] ionic liquid' TETRAHEDRON LETTERS vol. 45, 2004, pages 4673 - 4676, XP004509558**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[Technical Field]

The present invention relates to a method of preparing organic acids from aldehyde compounds by means of liquid phase oxidation using an ionic liquid, and more precisely, a method of preparing organic acids by liquid phase oxidation which converts aldehyde groups of those compounds harboring one or more aldehyde groups into carboxyl groups by using an ionic liquid as a reaction solvent or reaction catalyst.

[Background Art]

Preparation of organic acids by means of liquid phase oxidation is a well-known process. The conventional techniques and processes for the preparation of organic acids are described in WO 99/054274, US Patent No. 4,487,720, Japanese Patent No. 53-105413. According to the descriptions, oxidation of an aldehyde compound progresses with oxygen or air in the presence or absence of a catalyst. The oxidation can be carried out in the gas phase, but liquid phase oxidation is more common.

Regardless of the presence or absence of a catalyst, percarboxylic acid is produced as a reaction intermediate, and aldehyde oxidation is mainly achieved in a stainless steel reactor but it is sometimes carried out in a glass- or enamel-coated reactor.

When the oxidation has been carried out in the presence of a catalyst, according to the earlier reports, salts of transition metals, alkaline metals or alkaline earth metals have been used for the liquid phase oxidation of aldehyde compounds to produce corresponding organic acids. On the contrary, when the reaction has been carried out in the absence of a catalyst, a specially designed reactor and techniques thereof have been required for the efficient reaction between an aldehyde compound and oxygen (US Patent No. 4,350,829, Japanese Patent Nos. 53-108915, 53-13223, 53-13225 and 55-17131, and European Patent No. 1073621).

As aldehyde oxidation progresses, sub-reactions occur and various byproducts are generated in addition to a target organic acid, resulting in a lower yield. To obtain a pure organic acid, such byproducts have to be separated from the final organic acid product, requiring an additional process. Besides, such byproducts are discarded as waste matters, suggesting industrial loss during the process and thereby a low yield. Thus, it is important to minimize the generation of byproducts and maximize the yield of a reaction.

Japanese Patent No. 53-105413 describes an example of a conventional aldehyde oxidation reaction, wherein isobutyric acid and its isomer isopropylformate were produced from isobutylaldehyde by means of liquid phase oxidation. In the reaction, however, isopropylformate, a reaction byproduct, was not able to be easily separated because it had a similar boiling range as the main reactant isobutyraldehyde. Therefore, it is still necessary to reduce isopropylformate generation to achieve a high yield of isobutyric acid.

The Ionic liquid is composed of authentic ions only, and has been used as a reaction solvent or a reaction catalyst in a variety of chemical reactions. The techniques using the ionic liquid as a reaction catalyst have been described in US Patent Nos. 5,824, 832 and 5,731,101, and WO Nos. 00/015594 and 00/032572. The ionic liquid has advantages as a reaction solvent or a reaction catalyst in that it is a mixture of salts having a melting point up to 100°C, it exists in a liquid phase at room temperature, it is less volatile at high temperature and thermo-stable so a reaction may be carried out stably, it is easily separated from the reaction product upon completion of the reaction and can be reused, making it an excellent candidate for pro-environmental processes.

The ionic liquid, which is in a liquid phase at low temperature, has been proven to be a good reaction solvent or a reaction catalyst for such reactions as alkylation, polymerization, dimerization, oligomerization, acetylation, metatheses, hydrogenation and hydroformylation. However, there have been no examples reporting that such an ionic liquid is used as a reaction catalyst or a reaction solvent for oxidation, including aldehyde oxidation.

CN 1 544 410 A discloses a method for aldehyde oxidation to produce carboxylic esters using an oxidizing agent in the presence of an ionic liquid.

Howarth J.: "Oxidation of aromatic aldehydes in the ionic liquid [bmim]PF6", Tetrahedron Letters Vol. 41, 2000, pages 6627 - 6629, discloses a process for the oxidation of aromatic aldehydes using oxygen as the oxidizing agent in the presence of 1-butyl-3-methylimidazolium hexafluorophosphates.

[Disclosure of Invention]

It is an object of the present invention to provide a method of preparing organic acids from aldehyde compounds by means of liquid phase oxidation using an ionic liquid as a reaction solvent or a reaction catalyst, and thereby to increase the conversion rate of a reaction and selectivity, to maximize yield and to increase economic and environmental effects by separating the used ionic liquid from the reaction product and the recycling thereof.

The above object of the present invention and other additional objects can be achieved by the exemplary embodiments of the present invention described in detail hereinafter.

To achieve the above object, the present invention provides a method of preparing organic acids by liquid phase oxidation according to claim 1.

The method of preparing organic acids from aldehyde compounds, by means of liquid phase oxidation using an ionic liquid, of the present invention is described in detail hereinafter.

According to the present invention, organic acids are prepared from aldehyde compounds having one or more aldehyde groups by means of liquid phase oxidation, thereby converting the aldehyde groups into carboxyl groups by using an ionic liquid as a reaction solvent or a reaction catalyst.

The ionic liquid used in the present invention is represented by the neutral $A^+B^-$ formula composed of organic or inorganic cations generally indicated as $A^+$ and organic or inorganic anions generally indicated as $B^-$.

US Patent No. 5,731,101 or WO 95/021872 describes the method of preparing a neutral ionic liquid, and the cations and anions described in those are as follows:

The cations generally indicated as $A^+$ are exemplified by imidazoles, pyridines, pyrazoles, thiazoles, isothiazoles, azathiazoles, oxothiazoles, oxaines, oxazolines, oxazoboroles, dithiozoles, triazoles, selenozoles, oxaphospholes, pyrroles, boroles, furans, thiophenes, phospholes, pentazoles, indoles, indolines, oxazoles, isooxazoles, isotriazoles, tetrazoles, benzofurans, dibenzofurans, benzothiophenes, dibenzothiophenes, thiadiazoles, pyrimidines, pyrazines, pyridazines, piperazines, piperidines, morpholenes, pyrans, annolines, phthalzines, quinazolines, quinoxalines, quinolines, isoquinolines, thazines, oxazines or azaannulenes, from which one or more cations can be selected as the cation of the ionic liquid of the present invention. In addition, cation containing alkaline metals or alkaline earth metals or transition metals including Fe, Cu, Co, Mo, Rh, Pd or Pt can be used.

Among those cations, pyridinium or imidazolium cations are preferred and N-alkylpyridinium or N,N-dialkylimidazolium cations harboring a methyl group or butyl group as an alkyl group, to prevent the increase of viscosity and the decrease of yield caused by the increased sub-reaction, are more preferred. The formula is as follows.

**【Formula 1】**

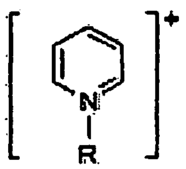

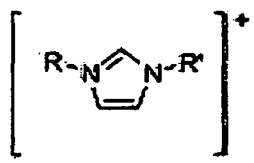

Wherein, R and R' are each independently $C_1$-$C_4$ alkyl.

The anions generally represented by $B^-$ are salts containing elements of the IB, IIIA, IVA, VIA or VIIA family on the periodic table or carboxyl group or halide salt-forming anions, and are exemplified by borates, phosphates, nitrates, sulfates, triflates, halogenized copperates, antimonates, carboranes, poly-oxo metallates, metalloboranes, and carboxylates or halides-forming anions. Among these anions, $BF_4^-$, $PF_6^-$, $CF_3SO_3^-$, $CF_3COO^-$, $SbF_6^-$, $[CuCl_2]^-$, $AsF_6^-$, $SO_4^-$, $CF_3CH_2CH_2COO^-$, $(CF_3SO_2)_3C^-$, $CF_3(CF_2)_3SO_3^-$, $[CF_3SO_2]_2N^-$ or inorganic anions are preferred and particularly $BF_4^-$ or $PF_6^-$ are more preferred. The ionic liquid of the present invention contains one or more anions selected from the above.

The ionic liquidis used together with a cocatalyst harboring an alkaline metal salt or alkaline earth metal salt as a major component. The anion in the alkaline metal salt or alkaline earth metal salt used as a cocatalyst can include one or more carboxyl groups, which is exemplified by formic acid, acetic acid, propionic acid, butyric acid, valeric acid, caproic acid, caprylic acid, capric acid, lauric acid, phenylacetic acid, benzoic acid, phthalic acid, isophthalic acid, terephthalic acid, adipic acid, 2-ethylhexanoic acid, isobutyric acid, 2-methylbutyric acid, 2-propylheptanoic acid, 2-phenylpropionic acid, 2-(p-isobutylphenyl)propionic acid or 2-(6-methoxy-2-naphthyl)propionic acid.

As a reaction solvent, the ionic liquid is characterized by the fact that it can contain alkaline metal salt or alkaline earth metal salt, which is a cocatalyst with a strong ionic property, selectivity at high concentration because of the strong ionic property in that liquid, and it can be easily separated and reused from the reaction product upon completion of the reaction.

The ionic liquid of the invention acts not only as a reaction solvent for the oxidation of aldehyde but also as a reaction catalyst.

It is preferred for the ionic liquid to be acidic for it to act as a reaction catalyst. An acidic ionic liquid is characterized by the fact that the cation therein is one of those neutral cations mentioned above whereas the anion therein contains a carboxyl group. The acidic ionic liquid can be prepared by a separate process from a cation of an alkaline metal or alkaline earth metal and a carboxylic acid such as formic acid, acetic acid, propionic acid, butyric acid, isobutyric acid

and hexoic acid or salts with the linkage of an anion of 2-ethylhexoic acid. That is, the acidic ionic liquid can be prepared by the reaction between salts containing a hydroxyl group or a carboxyl group and the neutral cation, then eliminating the reaction product alkaline metal salt or alkaline earth metal salt upon completion of the reaction. For example, imidazolium chloride is reacted with sodium acetate and then the produced sodium chloride is eliminated, resulting in an acidic ionic liquid, which is described in the exemplary embodiment below.

The advantages of the ionic liquid as an oxidation catalyst are as follows; it lowers the generation of byproducts to increase yield and it is easily separated from the reaction product upon completion of the reaction and reused, making the process efficient.

The compounds containing an aldehyde group used as a raw material for the present invention are generally prepared by hydroformylation. The purity of the aldehyde compound does not affect reactivity, but at least approximately 90%, and more preferably at least 95%, would be preferred. The compounds containing an aldehyde group have a formula of R-CHO, and R herein is preferably H or $C_1$-$C_8$ straight or branched alkyl. The aldehyde compound having more than $C_8$ exhibits low reactivity and yield owing to the bulky structure. The aldehyde compound is one or more compounds selected from a group consisting of compounds harboring formaldehyde, acetaldehyde, propionaldehyde, n-butyraldehyde, i-butyraldehyde, 2-methylbutyraldehyde, n-valealdehyde, caproaldehyde, heptaaldehyde, nonylaldehyde, phenylacetylaldehyde, benzaldehyde, o-tolualdehyde, m-tolualdehyde, p-tolualdehyde, salycylaldehyde, p-hydroxybenzaldehyde, anisaldehyde, vanilin, piperonal, 2-ethylhexylaldehyde, 2-propylheptaaldehyde, 2-phenylpropionaldehyde, 2-[p-isophenyl]propionaldehyde or 2-[6-methoxy-2-naphtyl]propionaldehyde.

The oxidation process of the aldehyde compound is explained in detail hereinafter.

First, for the liquid phase oxidation, oxygen in the air can be used but considering the efficiency of the reaction, oxygen or oxygen-rich air containing at least 50% oxygen is preferred.

The reaction pressure might be 0,1-10 MPa (normal pressure (1 atm) - 100 atm) 0,3-0,8 MPa (but 3-8 atm) is preferred. The reaction can be carried out at normal pressure but is preferably performed at a pressure to enhance oxygen solubility, resulting in high conversion rate and increased selectivity to organic acids.

The possible reaction temperature range is -20 to 150°C, but 10 to 120°C is preferred and 15 to 100°C is more preferred. If the reaction temperature is higher than 150°C, sub-reaction increases and thereby yield drops. On the contrary, if the reaction temperature is lower than -20°C, selectivity of organic acids might be increased but the oxygen content in the reaction system increases as well, risking stability. Thus, it is better to avoid driving the reaction at a temperature which is too low.

The reaction time depends on the amount of oxygen flowing in and the method of controlling heat. But, generally 15 minutes to 10 hours of reaction is preferred, 30 minutes to 8 hours of reaction is more preferred and 1 to 5 hours of reaction is most preferred. The reaction generates a large amount of heat, suggesting that control of the reaction heat is required. If the reaction heat is not controlled properly, it might explode. Up to 15 minutes of reaction time results in low yield and at least 10 hours of reaction time results in difficulty in controlling the reaction heat.

Considering the conversion rate, batch type reaction can be induced with a CSTR (continuous stirred tank reactor) for the liquid phase oxidation of the invention, but considering economical efficiency, it is more preferred to operate two consecutive CSTRs for a continuous type reaction. When two different CSTRs are operated consecutively, the reaction temperature might be set equally but to increase the conversion rate, it is preferred to increase the reaction temperature gradually.

In addition to the CSTR, a bubble column type reactor equipped with a gas distribution plate or a loop type recycled reactor equipped with a Venturi nozzle wherein gas and liquid are mixed can be effectively used for liquid phase oxidation.

According to the present invention, the ionic liquid can be easily separated from the reactant or product so that it can be reused, and precisely, the liquid phase mixture containing the ionic liquid can be easily separated from the reaction product upon completion of the liquid phase oxidation and the ionic liquid can be again separated from the liquid phase mixture for reuse. At this time, selective separation of the ionic liquid can be performed by distillation, washing or extraction.

According to the present invention, organic acids containing carboxylic acid such as formic acid, acetic acid, propionic acid, butyric acid, valeric acid, caproic acid, capryic acid, capric acid, lauric acid, phenylacetic acid, benzoic acid, phthalic acid, isophthalic acid, terephthalic acid, adipic acid, 2-ethylhexanoic acid, isobutyric acid, 2-methylbutyric acid, 2-propylheptanoic acid, 2-phenylpropionic acid, 2-(p-isobutylphenyl)propionic acid or 2-(6-methoxy-2-naphthyl) propionic acid can be prepared.

[Best Modes for Carrying out the Invention]

Practical and presently preferred embodiments of the present invention are illustrated as shown in the following Examples.

Example 1

The liquid phase oxidation of isobutylaldehyde was performed in a CSTR using the general ionic liquid 1-butyl-3-methyl imidazolium tetrafluoroborate ([bmim)$BF_4$). Particularly, to a 450 mℓ stainless steel reactor equipped with a cooling coil inside were added 200 g of isobutylaldehyde, 15g of the ionic liquid [bmim]$BF_4$ and 3 g of 50% KOH solution as a cocatalyst. The inside of the reaction system was purged with nitrogen and then the reaction temperature was raised to 50°C. The stirring speed was maintained at 1000 rpm. After stabilizing the reaction temperature, oxygen was injected at 150 mℓ/minute to start the reaction. The reaction pressure was slowly increased and when the final reaction pressure reached 8 atm, the reaction was terminated. Upon completion of the reaction, the reaction product was tested, and conversion rate and selectivity were calculated by the following formula giving the results shown in Table 1.

**[Mathematical Formula 1]**

**Conversion Rate = (mol of reacted aldehyde compound/mol of total provided aldehyde) x 100(%)**

**[Mathematical Formula 2]**

**Selectivity = (mol of generated carboxylic acid/mol of reacted aldehyde compound) X 100(%)**

Example 2

An experiment was performed in the same manner as described in Example 1 except that the ionic liquid 1-butyl-3-methyl imidazolium hexafluorophosphate ([bmim]$PF_6$) was used instead of [bmim]$BF_4$. The results are shown in Table 1. Substitution of [bmim]$BF_4$ with [bmim]$PF_6$ resulted in a lower conversion rate but higher selectivity.

Example 3

An experiment was performed in the same manner as described in Example 1 except that the ionic liquid [bmim]$PF_6$ was used as a solvent and 170 g of 2-ethylhexylaldehyde was used as a raw material. The results are shown in Table 1. As shown in Table 1, the conversion rate was similar to that of the isobutylaldehyde conversion reaction, but selectivity was decreased to 90.6%.

Comparative Example 1

An experiment was performed in the same manner as described in Example 3 except that the ionic liquid [bmim]$PF_6$ was used as a solvent and a cocatalyst was not added. The results are shown in Table 1. Selectivity was confirmed to be at a very low level of 76.2%.

Comparative Example 2

An experiment was performed in the same manner as described in Example 1 except that the ionic liquid was not used and 200 g of isobutylaldehyde was reacted with oxygen. The results are shown in Table 1. As shown in Table 1, the conversion rate was high but selectivity was low.

Comparative Example 3

An experiment was performed in the same manner as described in Comparative Example 2 except the ionic liquid was not used and 200 g of 2-ethylhexylaldehyde was used. Herein, the conversion rate was also high but selectivity was comparatively low.

[Table 1]

| | Solvent | Catalyst | Raw material | Conversion rate (%) | Selectivity (%) |
|---|---|---|---|---|---|
| Example 1 | $[bmim]BF_4$ | KOH | Isobutyl aldehyde | 99.2 | 93.5 |
| Example 2 | $[bmim]PF_6$ | KOH | Isobutyl aldehyde | 98.7 | 94.3 |
| Example 3 | $[bmim]PF_6$ | KOH | 2-ethylhexyl aldehyde | 98.5 | 90.6 |
| Comparative Example 1 | $[bmim]PF_6$ | - | 2-ethylhexyl aldehyde | 99.6 | 76.2 |
| Comparative Example 2 | - | KOH | Isobutylaldehyde | 99.8 | 86.2 |
| Comparative Example 3 | - | KOH | 2-ethylhexyl aldehyde | 99.4 | 84.6 |

Example 4

Two reactors which were the same as those used in Example 1 were serially installed. To each of the two reactors were added 420 mℓ of 2-ethylhexoic acid. The reactors were purged with nitrogen and the temperatures of the reactors were stabilized. 2-ethylhexylaldehyde solution mixed with the ionic liquid $[bmim]BF_4$ and KOH was injected into the first reactor at a rate of 3.4 mℓ/min. At this time, oxygen was injected to a level of 0.55, which is a little higher than the equivalence ratio (0.5) that is able to induce oxidation of 2-ethylhexylaldehyde. Most of the oxidation was carried out in the first reactor and then the reactant, the product and the ionic liquid containing solution were injected into the second reactor along with oxygen, wherein most of the oxygen was consumed to terminate the reaction. The reaction pressure of the first and the second reactor was set at 0,1 MPa (1 atm), the length of stay in each reactor was three hours and the stirring speed of a stirrer was maintained as 1,000 rpm. The reaction product was analyzed by gas-chromatography (GC) every three hours. The reaction product characteristically comprises two different layers; the ionic liquid layer and the reaction product layer. The reaction product layer was subjected to continuously flow out to the upper of the two separated layers and the lower ionic liquid layer was subjected to be reused in the reactors. Thus, after the first reaction was performed, the ionic liquid was not supplied any more but reused repeatedly in the reactors. Conversion rate and selectivity according to the liquid phase oxidation using such serial reactors are shown in Table 2.

Comparative Example 4

In this example, $[bmim]BF_4$ was used as a solvent and a bubble column reactor was used for the reaction instead of CSTR with two reactors consecutively connected. The bubble column reactor was equipped with a system necessary for cooling and heating the outside of the reactor. Reactant and reaction gas were passed through a filter plate located near the bottom, gathering in the lower part. The diameter of the filter is determined by the flow rate of the gas flowing into the reactor, and was approximately 16 - 40 micron. In the aspect of stability, the bubble column reactor is preferred since it does not require mixing. However, to increase conversion rate, one or more bubble columns have preferably been set up consecutively. The operating pressure was set at 0,1 MPa (1 atm) and details of the operating conditions were the same as described in Example 5. Conversion rate and selectivity resulting from the liquid phase oxidation are shown in Table 2.

Comparative Example 5

In this example, $[bmim]BF_4$ was used as a solvent and a loop type recycled reactor was used instead of a CSTR with two reactors connected serially. The loop type recycled reactor used herein, based on the general Venturi's principle, was equipped with a Venturi nozzle on the upper part, and negative pressure at the bottom of the Venturi was induced by the gas blowing out of the Venturi nozzle for the smooth gas flow-in. This reactor is characterized by the fact that gas flowing in through the Venturi tube and the reaction solution coming in through the pump set up outside of the reactor are consecutively circulated through the Venturi tube, suggesting that the mixing of liquid and gas is more convenient. Details of the operating conditions were as follows; the total volume of the reaction solution was 1 liter, reaction pressure was set at 0,1 MPa (1 atm), Venturi tube is 2 mm in diameter and circulating speed of the reaction solution was 1 liter/min. One reactor was used alone and the retention time of the reaction was three hours. And the operating conditions were the same as described in Example 4. Conversion rate and selectivity resulting from the liquid phase oxidation induced by the reaction of Example 6 are shown in Table 2.

[Table 2]

| | Solvent | Temp. of the first reactor (°C) | Temp. of the second reactor (°C) | Conversion rate (%) | Selectivity (%) |
|---|---|---|---|---|---|
| Comparative Example 4 | $[bmim]BF_4$ | 50 | 50 | 96.3 | 93.4 |
| Comparative Example 4 | $[bmim]BF_4$ | 50 | 50 | 97.2 | 92.5 |
| Comparative Example 5 | $[bmim]BF_4$ | 50 | - | 96.5 | 94.9 |

(Preparation of [bmim]OH)

The conventional $[bmin]PF_6$ was modified to prepare 1-butyl-3-methyl imidazolium hydroxide ([bmim]OH). 1-butyl-3-methyl imidazolium chloride (0.1 mol, 17.5 g), potassium hydroxide (0.1 mol, 5.6 g) and 50 ml of tetrahydrofuran (THF) were put in a 4-neck flask equipped with a reflux condenser on the upper part, followed by purging with nitrogen. The mixture was then stirred at room temperature for 12 hours. The reaction mixture was light brown at the early stage but later turned out dark brown. Upon completion of the reaction, pressure was reduced and the solvent was distilled, the precipitate was filtered, and finally the ionic liquid was separated and dried under the reduced pressure to give [bmim] OH.

(Preparation of [bmim] acetate)

The prepared [bmim]OH was reacted with acetic acid to prepare 1-butyl-3-methyl imidazolium acetate ([bmim] acetate). First, [bmim]OH (0.1 mol, 15.6 g) was mixed with acetic acid (0.1 mol, 9.8 g) in a 4-neck flask equipped with a reflux condenser on the upper part, followed by purging with nitrogen. The mixture was stirred at room temperature for 12 hours. Upon completion of the reaction, pressure was reduced ($10^{-2}$ torr), water was eliminated, the precipitate was filtered and finally the ionic liquid was separated under the reduced pressure to give [bmim]acetate.

Comparative Example 6

In this example, [bmim]OH was used as a reaction catalyst. Oxidation was induced with 200 g of isobutylaldehyde used as a raw material in the presence of 8,2 g of [bmim]OH. Addition of KOH was not required because the ionic liquid used as a catalyst was good enough not to require KOH. Detailed processes of the experiment were the same as described in Example 1. The results are shown in Table 3.

Comparative Example 7

In this example, [bmim]acetate was used as a catalyst. Detailed description of the liquid phase oxidation reaction was the same as described in Example 1 except that 14.3 g of [bmim]acetate was used for the oxidation. Addition of KOH was not required because the ionic liquid used as a catalyst was good enough not to require KOH. The results are shown in Table 3.

Comparative Example 8

In this example, [bmim]OH was used as a reaction catalyst. Oxidation was induced with 170 g of 2-ethylhexylaldehyde used as a raw material in the presence of 8,2 g of [bmim]OH. Addition of KOH was not required because the ionic liquid used as a catalyst was good enough not to require KOH. Detailed processes of the experiment were the same as described in Example 1. The results are shown in Table 3.

Comparative Example 9

In this example, [bmim]acetate was used as a catalyst. Detailed description of the liquid phase oxidation reaction was the same as described in Example 1 except that 14.3 g of [bmim]acetate was used for the oxidation. Addition of

KOH was not required because the ionic liquid used as a catalyst was good enough not to require KOH. The results are shown in Table 3.

[Table 3]

| | Solvent | Raw material | Conversion rate (%) | Selectivity (%) |
|---|---|---|---|---|
| Comparative Example 6 | [bmim]OH | Isobutylaldehyde | 99.2 | 96.7 |
| Comparative Example 7 | [bmim]acetate | Isobutylaldehyde | 99.5 | 97.1 |
| Comparative Example 8 | [bmim]OH | 2-ethylhexylaldehyde | 99.1 | 94.6 |
| Comparative Example 9 | [bmim]acetate | 2-ethylhexylaldehyde | 98.7 | 95.3 |

Comparative Example 10

The recirculation property of the ionic liquid acting as a catalyst was tested. Oxidation was induced under the same conditions as described in Comparative Example 9. Upon completion of the reaction, the ionic liquid was separated and a recirculation test was performed. Considering the subjects of this experiment, the ionic liquid was not 100% recovered and contained some by-products. Conversion rate was calculated based on the injected reactant. The results are shown in Table 4.

[Table 4]

| Circulation time | Conversion rate (%) | Selectivity (%) |
|---|---|---|
| 1 | 98.6 | 94.8 |
| 2 | 98.2 | 95.1 |
| 3 | 98.7 | 94.3 |
| 4 | 97.9 | 95.3 |

As shown in the examples, when oxidation was induced in the presence of the ionic liquid, a high conversion rate and selectivity of at least 90% were observed, unlike those from the oxidation without any ionic liquid. Even the recycled ionic liquid contributed to a high conversion rate and selectivity, indicating that using the ionic liquid is very economical and environmentally beneficial.

[Industrial Applicability]

As described hereinbefore, the present invention provides a method of using an ionic liquid as a reaction solvent or a reaction catalyst for the preparation of organic acids from aldehyde compounds by means of liquid phase oxidation. The ionic liquid is less volatile at high temperature and very stable against heat, suggesting great potential to enhance conversion rate and selectivity and resultingly increase the yield. The ionic liquid is also very advantageous since it can be easily separated from the reactant and product and reused, thereby reducing costs and wastewater.

Therefore, the method of preparing organic acids by liquid phase oxidation using an ionic liquid as a solvent or a catalyst, is less volatile and stable against heat at high temperature, can reduce the byproduct generation, means an increase in yield, and it can also be easily separated from the reactant and product so as to be recycled, reducing the production cost and waste matter.

## Claims

**1.** A method of preparing organic acids from aldehyde compounds, **characterized by** using an ionic liquid as a reaction solvent or a reaction catalyst for liquid phase oxidation to convert aldehyde groups of aldehyde compounds into carboxylic acid,
wherein the liquid phase oxidation is performed in a CSTR, a bubble column reactor or a loop type recycled reactor in the presence of air or oxygen, and
wherein an alkaline metal salt or alkaline earth metal salt is added as a cocatalyst for the liquid phase oxidation.

**2.** The method of preparing organic acids according to claim 1, wherein the ionic liquid is a neutral ionic liquid composed of cations and anions.

**3.** The method of preparing organic acids according to claim 2, wherein the cation is one or more cations selected from a group consisting of cations of imidazoles, pyridines, pyrazoles, thiazoles, isothiazoles, azathiazoles, oxothiazoles, oxaines, oxazolines, oxazoboroles, dithiozoles, triazoles, selenozoles, oxaphospholes, pyrroles, boroles, furans, thiophenes, phospholes, pentazoles, indoles, indolines, oxazoles, isooxazoles, isotriazoles, tetrazoles, benzofurans, dibenzofurans, benzothiophenes, dibenzothiophenes, thiadiazoles, pyrimidines, pyrazines, pyridazines, piperazines, piperidines, morpholenes, pyrans, annolines, phthalzines, quinazolines, quinoxalines, quinolines, isoquinolines, thazines, oxazines and azaannulenes.

**4.** The method of preparing organic acids according to claim 2, wherein the cation is a cation of an alkaline metal, alkaline earth metal or transition metal.

**5.** The method of preparing organic acids according to claim 4, wherein the transition metal is Fe, Cu, Co, Mo, Rh, Pd or Pt.

**6.** The method of preparing organic acids according to claim 2, wherein the cation is a cation of N-alkylpyridinium or N,N-dialkylimidazolium having the structural formula below:

[Formula 1]

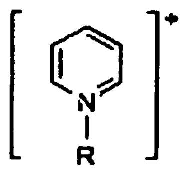

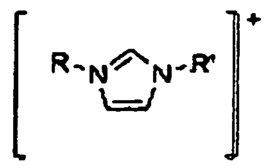

wherein, R and R' are each independently $C_1$-$C_4$ alkyl.

**7.** The method of preparing organic acids according to claim 2, wherein the anion is an anion of borates, phosphates, nitrates, sulfates, triflates, halogenized copperates, antimonates, carboranes, poly-oxo metallates, metalloboranes, carboxylates or halides.

**8.** The method of preparing organic acids according to claim 2, wherein the anion is one or more anions selected from a group consisting of $BF_4^-$, $PF_6^-$, $CF_3SO_3^-$, $CF_3COO^-$, $SbF_6^-$, $[CuCl_2]^-$, $AsF_6^-$, $SO_4^-$, $CF_3CH_2CH_2COO^-$, $(CF_3SO_2)_3C^-$, $CF_3(CF_2)_3SO_3^-$, $[CF_3SO_2]_2N^-$ and inorganic anions.

**9.** The method of preparing organic acids according to claim 1, wherein the anion of an alkaline metal salt or alkaline earth metal salt contains one or more carboxyl groups selected from a group consisting of formic acid, acetic acid, propionic acid, butyric acid, valeric acid, caproic acid, caprylic acid, capric acid, lauric acid, phenylacetic acid, benzoic acid, phthalic acid, isophthalic acid, terephthalic acid, adipic acid, 2-ethylhexanoic acid, isobutyric acid, 2-methylbutyric acid, 2-propylheptanoic acid, 2-phenylpropionic acid, 2-(p-isobutylphenyl)propionic acid and 2-(6-methoxy-2-naphthyl)propionic acid.

**10.** The method of preparing organic acids according to claim 1, wherein the reaction catalyst is an acidic ionic liquid.

**11.** The method of preparing organic acids according to claim 10, wherein the cation of the acidic ionic liquid includes the cation of claim 2 and the anion includes a carboxyl group.

**12.** The method of preparing organic acids according to claim 1, wherein the aldehyde group containing compound is one or more compounds selected from a group consisting of formaldehyde, acetaldehyde, propionaldehyde, n-butyraldehyde, i-butyraldehyde, 2-methylbutyraldehyde, n-valealdehyde, caproaldehyde, heptaaldehyde, nonylaldehyde, phenylacetylaldehyde, benzaldehyde, o-tolualdehyde, m-tolualdehyde, p-tolualdehyde, salacylaldehyde,

p-hydroxybenzaldehyde, anisaldehyde. vanillin, piperonal, 2-ethylhexylalehyde, 2-propylheptaaldehyde, 2-phenylpropionaldehyde, 2-[p-isophenyl]propionaldehyde and 2-[6-methoxy-2-naphthyl]propionaldehyde.

**13.** The method of preparing organic acids according to claim 1, wherein the liquid phase oxidation is induced in the presence of air or oxygen under a pressure of 0,1 - 10 MPa (1 - 100 atm), at -20 - 150°C, for 15 minutes - 10 hours.

**14.** The method of preparing organic acids according to claim 1, wherein the liquid phase oxidation is performed as a batch type reaction using one CSTR or a continuous reaction using at least two CSTRs stationed serially.

**15.** The method of preparing organic acids according to claim 14, wherein the temperatures of at least two reactors used for the continuous reaction are equally maintained or increased gradually.

**16.** The method of preparing organic acids according to claim 1, wherein the ionic liquid containing liquid mixture is separated from the reactant upon completion of the reaction, and then the ionic liquid is separated again from the liquid mixture separated above, and is then ready to be reused.

**17.** The method of preparing organic acids according to claim 16, wherein the separation of the ionic liquid is performed by distillation, washing, or extraction.

**18.** The method of preparing organic acids according to any one of claims 1-17, wherein the organic acid is one or more compounds selected from a group consisting of formic acid, acetic acid, propionic acid, butyric acid, valeric acid, caproic acid, caprylic acid, capric acid, lauric acid, phenylacetic acid, benzoic acid, phthalic acid, isophthalic acid, terephthalic acid, adipic acid, 2-ethylhexanoic acid, isobutyric acid, 2-methylbutyric acid, 2-propylheptanoic acid, 2-phenylpropionic acid, 2-(p-isobutylphenyl)propionic acid and 2-(6-methoxy-2-naphthyl)propionic acid.

## Patentansprüche

**1.** Verfahren zur Herstellung von organischen Säuren aus Aldehydverbindungen, **gekennzeichnet durch** Verwendung einer ionischen Flüssigkeit als einem Reaktionslösemittel oder einem Reaktionskatalysator für Flüssigphasenoxidation, um Aldehydgruppen von Aldehydverbindungen in Carbonsäure umzuwandeln,
wobei die Flüssigphasenoxidation in einem CSTR, einem Blasensäulenreaktor oder einem Schlaufenkreislaufreaktor in Gegenwart von Luft oder Sauerstoff durchgeführt wird und
wobei ein Alkalimetallsalz oder Erdalkalimetallsalz als ein Cokatalysator für die Flüssigphasenoxidation zugesetzt wird.

**2.** Verfahren zur Herstellung von organischen Säuren nach Anspruch 1, wobei die ionische Flüssigkeit eine neutrale ionische Flüssigkeit ist, bestehend aus Kationen und Anionen.

**3.** Verfahren zur Herstellung von organischen Säuren nach Anspruch 2, wobei das Kation ein oder mehrere Kationen ist, die ausgewählt sind aus einer Gruppe, bestehend aus Kationen von Imidazolen, Pyridinen, Pyrazolen, Thiazolen, Isothiazolen, Azathiazolen, Oxothiazolen, Oxainen, Oxazolinen, Oxazoborolen, Dithiozolen, Triazolen, Selenozolen, Oxaphospholen, Pyrrolen, Borolen, Furanen, Thiophenen, Phospholen, Pentazolen, Indolen, Indolinen, Oxazolen, Isooxazolen,
Isotriazolen, Tetrazolen, Benzofuranen, Dibenzofuranen, Benzothiophenen, Dibenzothiophenen, Thiadiazolen, Pyrimidinen, Pyrazinen, Pyridazinen, Piperazinen, Piperidinen, Morpholenen, Pyranen, Annolinen, Phthalzinen, Chinazolinen, Chinoxalinen, Chinolinen, Isochinolinen, Thazinen, Oxazinen und Azaannulenen.

**4.** Verfahren zur Herstellung von organischen Säuren nach Anspruch 2, wobei das Kation ein Kation eines Alkalimetalls, Erdalkalimetalls oder Übergangsmetalls ist.

**5.** Verfahren zur Herstellung von organischen Säuren nach Anspruch 4, wobei das Übergangsmetall Fe, Cu, Co, Mo, Rh, Pd oder Pt ist.

**6.** Verfahren zur Herstellung von organischen Säuren nach Anspruch 2, wobei das Kation ein Kation von N-Alkylpyridinium oder N,N-Dialkylimidazolium ist, mit der Strukturformel unten:

[Formel 1]

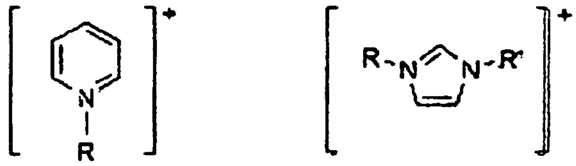

,

wobei R und R' jeweils unabhängig $C_1$-$C_4$-Alkyl sind.

**7.** Verfahren zur Herstellung von organischen Säuren nach Anspruch 2, wobei das Anion ein Anion von Boraten, Phosphaten, Nitraten, Sulfaten, Triflaten, halogenisierten Copperaten, Antimonaten, Carboranen, Polyoxometallaten, Metalloboranen, Carboxylaten oder Halogeniden ist.

**8.** Verfahren zur Herstellung von organischen Säuren nach Anspruch 2, wobei das Anion ein oder mehrere Anionen ist, die ausgewählt sind aus einer Gruppe, bestehend aus $BF_4^-$, $PF_6^-$, $CF_3SO_3^-$, $CF_3COO^-$, $SbF_6^-$, $[CuCl_2]^-$, $AsF_6^-$, $SO_4^-$, $CF_3CH_2CH_2COO^-$, $(CF_3SO_2)_3C^-$, $CF_3(CF_2)_3SO_3^-$, $[CF_3SO_2]_2N^-$ und anorganischen Anionen.

**9.** Verfahren zur Herstellung von organischen Säuren nach Anspruch 1, wobei das Anion eines Alkalimetallsalzes oder Erdalkalimetallsalzes eine oder mehrere Carboxylgruppen enthält, die ausgewählt sind aus einer Gruppe, bestehend aus Ameisensäure, Essigsäure, Propionsäure, Buttersäure, Valeriansäure, Capronsäure, Caprylsäure, Caprinsäure, Laurinsäure, Phenylessigsäure, Benzoesäure, Phthalsäure, Isophthalsäure, Terephthalsäure, Adipinsäure, 2-Ethylhexansäure, Isobuttersäure, 2-Methylbuttersäure, 2-Propylheptansäure, 2-Phenylpropionsäure, 2-(p-Isobutylphenyl)propionsäure und 2-(6-Methoxy-2-naphthyl)propionsäure.

**10.** Verfahren zur Herstellung von organischen Säuren nach Anspruch 1, wobei der Reaktionskatalysator eine saure ionische Flüssigkeit ist.

**11.** Verfahren zur Herstellung von organischen Säuren nach Anspruch 10, wobei das Kation der sauren ionischen Flüssigkeit das Kation von Anspruch 2 einschließt und das Anion eine Carboxylgruppe einschließt.

**12.** Verfahren zur Herstellung von organischen Säuren nach Anspruch 1, wobei die eine Aldehydgruppe enthaltende Verbindung eine oder mehrere Verbindungen ist, die ausgewählt sind aus einer Gruppe, bestehend aus Formaldehyd, Acetaldehyd, Propionaldehyd, n-Butyraldehyd, i-Butyraldehyd, 2-Methylbutyraldehyd, n-Valealdehyd, Caproaldehyd, Heptaaldehyd, Nonylaldehyd, Phenylacetylaldehyd, Benzaldehyd, o-Tolualdehyd, m-Tolualdehyd, p-Tolualdehyd, Salicylaldehyd, p-Hydroxybenzaldehyd, Anisaldehyd, Vanillin, Piperonal, 2-Ethylhexylaldehyd, 2-Propylheptaaldehyd, 2-Phenylpropionaldehyd, 2-[p-Isophenyl]propionaldehyd und 2-[6-Methoxy-2-naphthyl]propionaldehyd.

**13.** Verfahren zur Herstellung von organischen Säuren nach Anspruch 1, wobei die Flüssigphasenoxidation in Gegenwart von Luft oder Sauerstoff unter einem Druck von 0,1 - 10 MPa (1 - 100 atm), bei -20 - 150°C, für 15 Minuten - 10 Stunden induziert wird.

**14.** Verfahren zur Herstellung von organischen Säuren nach Anspruch 1, wobei die Flüssigphasenoxidation als eine Batch-Reaktion unter Verwendung eines CSTRs oder eine kontinuierliche Reaktion unter Verwendung von wenigstens zwei CSTRs, die in Reihe geschaltet sind, durchgeführt wird.

**15.** Verfahren zur Herstellung von organischen Säuren nach Anspruch 14, wobei die Temperaturen von wenigstens zwei für die kontinuierliche Reaktion verwendeten Reaktoren gleichgehalten oder allmählich angehoben werden.

**16.** Verfahren zur Herstellung von organischen Säuren nach Anspruch 1, wobei die eine ionische Flüssigkeit enthaltende Flüssigkeitsmischung vom Reaktanten nach Abschluss der Reaktion abgetrennt wird und dann die ionische Flüssigkeit erneut von der oben abgetrennten Flüssigkeitsmischung abgetrennt wird und dann fertig zur Wiederverwendung ist.

17. Verfahren zur Herstellung von organischen Säuren nach Anspruch 16, wobei die Abtrennung der ionischen Flüssigkeit durch Destillation, Waschen oder Extraktion durchgeführt wird.

18. Verfahren zur Herstellung von organischen Säuren nach einem der Ansprüche 1 bis 17, wobei die organische Säure eine oder mehrere Verbindungen ist, die ausgewählt sind aus einer Gruppe, bestehend aus Ameisensäure, Essigsäure, Propionsäure, Buttersäure, Valeriansäure, Capronsäure, Caprylsäure, Caprinsäure, Laurinsäure, Phenylessigsäure, Benzoesäure, Phthalsäure, Isophthalsäure, Terephthalsäure, Adipinsäure, 2-Ethylhexansäure, Isobuttersäure, 2-Methylbuttersäure, 2-Propylheptansäure, 2-Phenylpropionsäure, 2-(p-Isobutylphenyl)propionsäure und 2-(6-Methoxy-2-naphthyl)propionsäure.

## Revendications

1. Procédé de préparation d'acides organiques à partir de composés d'aldéhyde, **caractérisé par** l'utilisation d'un liquide ionique comme solvant de réaction ou d'un catalyseur de réaction pour une oxydation en phase liquide afin de convertir des groupes aldéhydes de composés aldéhydes en acide carboxylique,
dans lequel l'oxydation en phase liquide s'effectue dans un CSTR, un réacteur de type colonne à bulles ou un réacteur recyclé de type à boucle en présence d'air ou d'oxygène, et
dans lequel un sel métallique alcalin ou un sel métallique alcalino-terreux est ajouté à titre de cocatalyseur pour l'oxydation en phase liquide.

2. Procédé de préparation d'acides organiques selon la revendication 1, dans lequel le liquide ionique est un liquide ionique neutre composé de cations et d'anions.

3. Procédé de préparation d'acides organiques selon la revendication 2, dans lequel le cation est un ou plusieurs des cations sélectionné(s) parmi le groupe constitué de cations d'imidazoles, pyridines, pyrazoles, thiazoles, isothiazoles, azathiazoles, oxothiazoles, oxaines, oxazolines, oxazoboroles, dithiozoles, triazoles, sélénozoles, oxaphospholes, pyrroles, boroles, furanes, thiophènes, phospholes, pentazoles, indoles, indolines, oxazoles, isooxazoles, isotriazoles, tétrazoles, benzofuranes, dibenzofuranes, benzothiophènes, dibenzothiophènes, thidiazoles, pyrimidines, pyrazines, pyridazines, pipérazines, pipéridines, morpholènes, pyranes, annolines, phtalzines, quinazolines, quinoxalines, quinolines, isoquinolines, thazines, oxazines et azaannulènes.

4. Procédé de préparation d'acides organiques selon la revendication 2, dans lequel le cation est un cation d'un métal alcalin, d'un métal alcalino-terreux ou d'un métal de transition.

5. Procédé de préparation d'acides organiques selon la revendication 4, dans lequel le métal de transition est Fe, Cu, Co, Mo, Rh, Pd ou Pt.

6. Procédé de préparation d'acides organiques selon la revendication 2, dans lequel le cation est un cation de N-alkylpyridinium ou de N,N-diakylimidazolium ayant la formule structurale figurant ci-dessous :

[Formule 1]

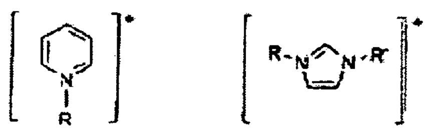

dans laquelle R et R' représentent chacun indépendamment un alkyle en $C_1$ à $C_4$.

7. Procédé de préparation d'acides organiques selon la revendication 2, dans lequel l'anion est un anion de borates, phosphates, nitrates, sulfates, triflates, coppérates halogénéisés, antimonates, carboranes, polyoxo-métallates, métalloboranes, carboxylates ou halides.

8. Procédé de préparation d'acides organiques selon la revendication 2, dans lequel l'anion est un ou plusieurs anions

sélectionné(s) parmi un groupe constitué de $BF_4^-$, $PF_6^-$, $CF_3SO_3^-$, $CF_3COO^-$, $SbF_6^-$, $[CuCl_2]^-$, $AsF_6^-$, $SO_4^-$, $CF_3CH_2CH_2COO^-$, $(CF_3SO_2)_3C^-$, $CF_3(CF_2)_3SO_3^-$, $[CF_3SO_2]_2N^-$ et des anions inorganiques.

**9.** Procédé de préparation d'acides organiques selon la revendication 1, dans lequel l'anion d'un sel métallique alcalin ou d'un sel métallique alcalino-terreux contient un ou plusieurs groupes carboxyle(s) sélectionné(s) parmi un groupe constitué d'acide formique, acide acétique, acide propionique, acide butyrique, acide valérique, acide caproïque, acide caprylique, acide caprique, acide laurique, acide phénylacétique, acide benzoïque, acide phtalique, acide isophtalique, acide téréphtalique, acide adipique, acide 2-éthylhexanoïque, acide isobutyrique, acide 2-méthylbutyrique, acide 2-propylheptanoïque, acide 2-phénylpropionique, acide 2-(p-isobutyle phényle)propionique et acide 2-(6-méthoxy-2-naphtyle)propionique.

**10.** Procédé de préparation d'acides organiques selon la revendication 1, dans lequel le catalyseur de réaction est un liquide ionique acidique.

**11.** Procédé de préparation d'acides organiques selon la revendication 10, dans lequel le cation du liquide ionique acidique renferme le cation de la revendication 2 et l'anion renferme un groupe carboxylique.

**12.** Procédé de préparation d'acides organiques selon la revendication 1, dans lequel le composé contenant le groupe aldéhyde est un ou plusieurs composés sélectionné(s) parmi un groupe constitué de formaldéhyde, acétaldéhyde, propionaldéhyde, n-butylraldéhyde, i-butyraldéhyde, 2-méthylbutyraldéhyde, n-valéaldéhyde, caproaldéhyde, heptaaldéhyde, nonylaldéhyde, phénylacétylaldéhyde, benzaldéhyde, o-tolualdéhyde, m-tolualdéhyde, p-tolualdéhyde, salicylaldéhyde, p-hydroxybenzaldéhyde, anisaldéhyde, vanilline, pipéronal, 2-éthylhexylaldéhyde, 2-propylheptaadéhyde, 2-phénylpropionaldéhyde, 2-[p-isophényle]propionaldéhyde et 2 -[6-méthoxy-2-naphthyle]propionaldéhyde.

**13.** Procédé de préparation d'acides organiques selon la revendication 1, dans lequel l'oxydation en phase liquide est induite en présence d'air ou d'oxygène sous une pression de 0,1 à 10 Mpa (1 à 100 atm) à -20 -150°C pendant 15 minutes - 10 heures.

**14.** Procédé de préparation d'acides organiques selon la revendication 1, dans lequel l'oxydation en phase liquide est effectuée en réaction de type en lots en utilisant un CSTR ou une réaction continue faisant appel à au moins deux CSTR positionnés en série.

**15.** Procédé de préparation d'acides organiques selon la revendication 14, dans lequel les températures d'au moins deux réacteurs utilisés pour la réaction continue sont maintenues constantes ou augmentées progressivement.

**16.** Procédé de préparation d'acides organiques selon la revendication 1, dans lequel le liquide ionique contenant le mélange liquide est séparé du réactif après l'achèvement de la réaction et le liquide ionique est ensuite séparé à nouveau du mélange liquide séparé ci-dessus et est ensuite prêt à être réutilisé.

**17.** Procédé de préparation d'acides organiques selon la revendication 16, dans lequel la séparation du liquide ionique est effectuée par distillation, lavage ou extraction.

**18.** Procédé de préparation d'aides organiques selon l'une quelconque des revendications 1 à 17, dans lequel l'acide organique est un ou plusieurs composés sélectionné(s) parmi un groupe constitué d'acide formique, acide acétique, acide propionique, acide butyrique, acide valérique, acide caproïque, acide caprylique, acide caprique, acide laurique, acide phénylacétique, acide benxoïque, acide phtalique, acide isophtalique, acide téréphtalique, acide adipique, acide 2-éthylhexanoïque, acide isobutyrique, acide 2-méthylbutyrique, acide 2-propylheptanoïque, acide 2-phénylpropionique, acide 2-(p-isobutylephényle(propionique et acide 2-(6-métyhoxy-2-naphtyle)propionique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 99054274 A
- US 4487720 A
- JP 53105413 A
- US 4350829 A
- JP 53108915 A
- JP 53013223 A
- JP 53013225 A
- JP 55017131 A
- EP 1073621 A
- US 5824832 A
- US 5731101 A
- WO 00015594 A
- WO 00032572 A
- CN 1544410 A
- WO 95021872 A


### Non-patent literature cited in the description


- **HOWARTH J.** Oxidation of aromatic aldehydes in the ionic liquid [bmim]PF6. *Tetrahedron Letters,* 2000, vol. 41, 6627-6629